# EUROPEAN PATENT SPECIFICATION

(11) **EP 3 025 668 B1**
(45) Date of publication and mention of the grant of the patent: **30.08.2017**
(21) Application number: 15193677.0
(22) Date of filing: 09.11.2015
(51) Int. Cl.: A61B 50/20, A61B 90/11, A61B 90/50, F16C 11/06, F16C 11/10, F16C 39/04, A61B 17/00

(54) **TOOL HOLDING ARTICULATED ARM**
GELENKIGER WERKZEUGHALTEARM
BRAS ARTICULÉ DE SUPPORT D'OUTIL

(30) Priority: 28.11.2014 JP 2014241106
(43) Date of publication of application: 01.06.2016
(73) Proprietor: MITAKA KOHKI CO., LTD., Tokyo 181-0014 (JP)
(72) Inventor: NAKATA, Yusuke, Tokyo 181-0014 (JP)
(74) Representative: Grünecker Patent- und Rechtsanwälte PartG mbB

(56) References cited:
- DE-A1- 19 526 915
- US-A1- 2007 282 311
- US-B1- 6 767 153

## Description

### BACKGROUND OF THE INVENTION

### 1. Field of the Invention

The present invention relates to a tool holding articulated arm.

### 2. Description of Related Art

Medical practice involves a variety of medical tools . For example, brain surgery needs a tool called a brain retractor. The brain retractor is used in brain surgery to partly push the brain and make a surgical part easily visible. Tools also used in medical field are a suction tube, an ultrasonic echo probe, an endoscope, and the like.

To hold such a medical tool at a required position, a tool holding articulated arm is used. The tool holding articulated arm has a plurality of joints that are freely flexed and fixed at given angles and positions.

An example of the tool holding articulated arm is disclosed in Japanese Patent No. 2843507(Patent Literature 1). This related art forms each joint of the tool holding articulated arm with a hollow inner sphere and a hollow outer sphere that are coupled with each other so that they may slide relative to each other. The related art provides one of the inner and outer spheres with a clutch that is pressed to the other sphere so as to establish a fixed state of the joint. Patents DE19526915 A1, US6767153 B1, and US2007282311 A1 also disclose tool holding devices. DE19526915 discloses the preamble of appended claim 1. The invention is defined by independent claim 1. Preferred embodiments are given in the dependent claims.

### SUMMARY OF THE INVENTION

According to the related art, the clutch arranged on one of the inner and outer spheres of each joint is pressed to the other sphere to make the joint immovable. This configuration needs a large operating force to be exerted on the clutch to establish the fixed state of the joint, and therefore, involves a complicated structure.

In consideration of the related art, the present invention provides a tool holding articulated arm that is capable of establishing a large immobilizing force with a small operating force.

According to a first aspect of the present invention, the tool holding articulated arm includes a plurality of joints. Each of the joints includes a ball having a spherical surface, a housing that accommodates the ball and has a front opening whose diameter is smaller than the diameter of the ball and a stopper bottom opposing the front opening, and a presser that is arranged between the stopper bottom and the ball and is moved toward and away from the ball by a pressing unit. The tool holding articulated arm also includes a tool holder that is to hold a tool and is attached to the ball of a foremost one of the plurality of joints that are connected in series with corresponding ends of the housing and ball of the adjacent joints being connected to each other.

According to a second aspect of the present invention, the presser of each joint of the tool holding articulated arm has a cup shape having a circumferential edge that is brought into linear contact with the surface of the ball of the joint.

According to a third aspect of the present invention, the pressing unit of each joint of the tool holding articulated arm includes a spring arranged between the stopper bottom and the presser to bias the presser toward the ball, an acting member coupled with a part of the presser behind the stopper bottom, and a sealed chamber defined between the acting member and the stopper bottom. When a gas is supplied into the sealed chamber, the acting member moves together with the presser away from the ball.

According to a fourth aspect of the present invention, the tool holding articulated arm also includes a double pipe that is flexible and is passed through the plurality of joints. The double pipe has an inner passage and an outer passage so that a compressed gas is supplied in one direction into the inner passage, is turned at an end of the inner passage into the outer passage, and is guided in the opposite direction through the outer passage . A valve is arranged at the end of the inner passage, to open and close the flow of the compressed gas. In each of the plurality of joints, a branch passage is branched from the outer passage and connected to the sealed chamber.

### BRIEF DESCRIPTION OF THE DRAWINGS

Figure 1 is a side view illustrating a tool holding articulated arm according to an embodiment of the present invention;
Fig. 2 is a perspective view partly illustrating a flexed state of the tool holding articulated arm;
Fig. 3 is a partly sectioned view illustrating a flexed state of the tool holding articulated arm;
Fig. 4 is a sectional view illustrating a joint of the tool holding articulated arm;
Fig. 5 is a sectional view illustrating the joint of the tool holding articulated arm with a ball of the joint being tilted to a flexed position;
Fig. 6 is an exploded sectional view illustrating a presser and the ball in the joint of the tool holding articulated arm;
Fig. 7 is an exploded perspective view illustrating the presser and ball of Fig. 6;
Fig. 8 is a sectional view illustrating a sealed chamber defined in the joint of the tool holding articulated arm;
Fig. 9 is a sectional view illustrating a gas supplied state of the sealed chamber of Fig. 8; and
Fig. 10 is a sectional view according to the invention illustrating a double pipe arranged through the joints of the tool holding articulated arm.

### DESCRIPTION OF PREFERRED EMBODIMENTS

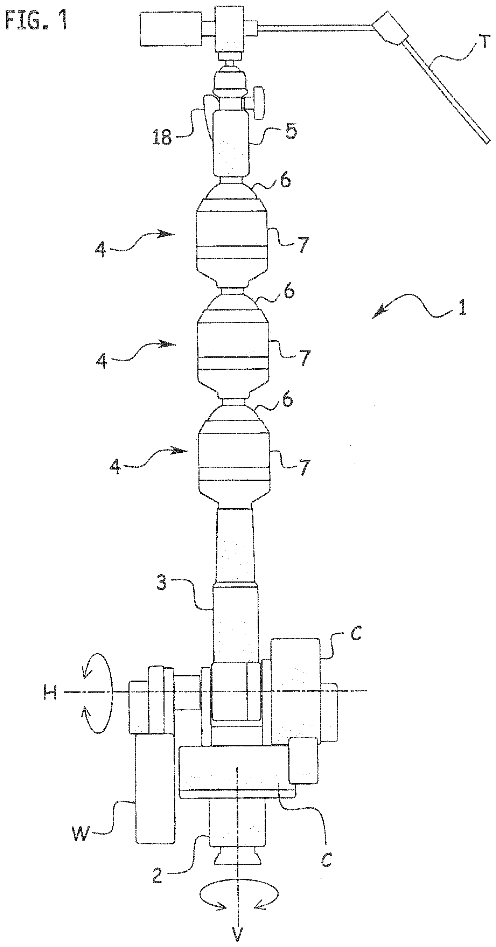

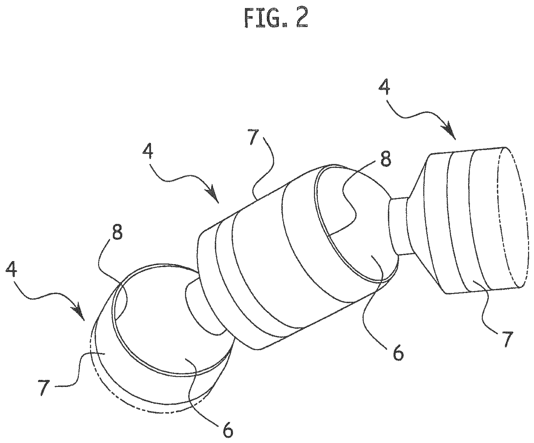

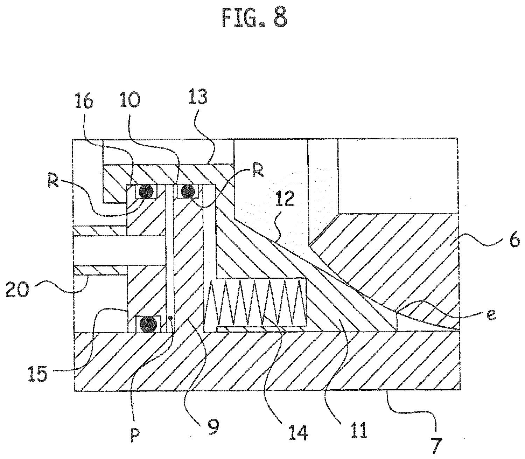

A tool holding articulated arm according to an embodiment of the present invention will be explained with reference to Figs. 1 to 10.

Figure 1 illustrates the tool holding articulated arm 1 according to the embodiment. In Fig. 1, the arm 1 is in an upright state and has a securing part 2 that is at a lower end of the arm 1 and is fixed to, for example, a side rail of an operating table in a hospital.

With respect to the securing part 2, the tool holding articulated arm 1 is horizontally rotatable around a vertical axis V and vertically rotatable around a horizontal axis H. On each of the vertical axis V and horizontal axis H, a clutch C is arranged to fix the arm 1 at an optional rotation angle around the axis. Also arranged on the horizontal axis H is a counterweight W that extends along the securing part 2.

The tool holding articulated arm 1 includes a base arm 3 at a proximal side thereof, three joints as articulations 4 connected in series, and a front tool holder 5 at a distal side thereof. The tool holder 5 holds a medical tool such as an endoscope T.

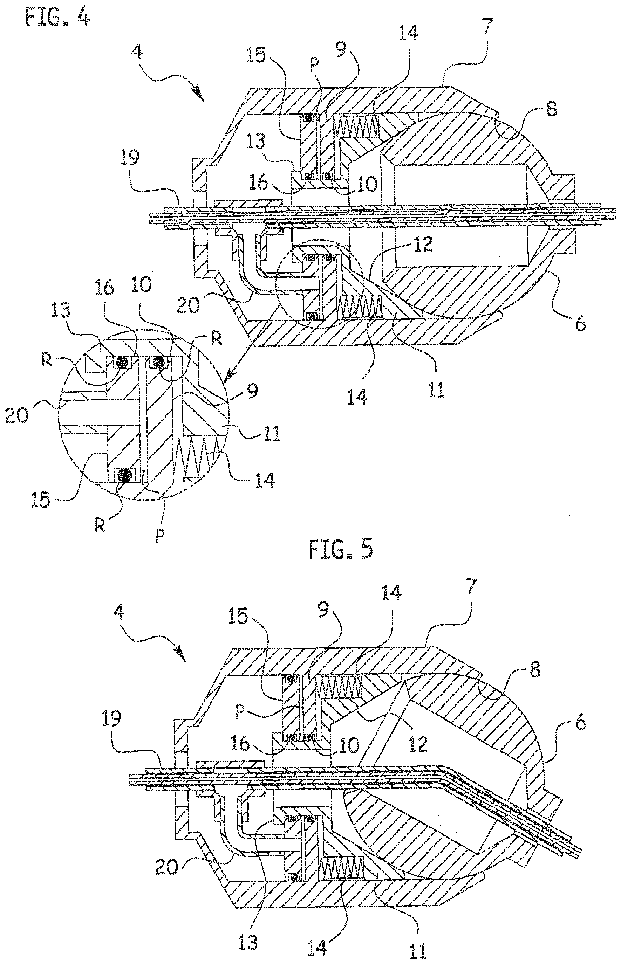

The articulations 4 each has the same structure, and therefore, the structure of one of the articulations 4 will be explained with reference to Fig. 4.

The articulation 4 is made of surface-treated aluminum alloy. The articulation 4 includes a ball 6 that has a spherical surface and a central through hollow that substantially has a cylindrical shape having a predetermined diameter. The ball 6 is contained in a housing 7 that basically has a cylindrical shape having a central through hole. A front opening 8 is formed at a front end of the housing 7. A base end of the housing 7 is narrowed.

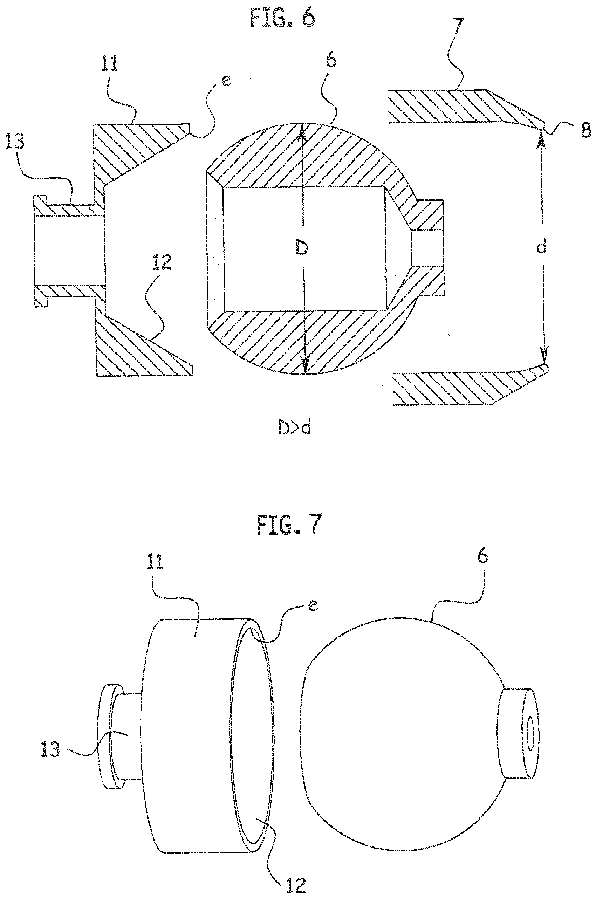

The front opening 8 of the housing 7 has an inner diameter d that is slightly smaller than a diameter *D* of the ball 6 as illustrated in Fig. 6 so that a part of the ball 6 may protrude outside from the front opening 8.

Inside the housing 7, there is formed a stopper bottom 9 having a center hole 10 that is provided with a sealing member such as an O-ring R. The O-ring R is illustrated as a black circle in the drawings. Between the stopper bottom 9 and the ball 6, the housing 7 accommodates a presser 11.

Although the housing 7 is illustrated as a single structure in the drawings, the housing 7 is actually made of a plurality of parts that are assembled into one with the ball 6, presser 11, and the like being incorporated inside the housing 7.

The presser 11 has a cup shape having a tapered face 12 or a conical frustum inner face. A base end of the presser 11 is a cylindrical presser end 13 that is in contact with the O-ring R of the center hole 10 of the stopper bottom 9. The presser 11 includes pressing means such as four springs 14 arranged at intervals in a circumferential direction. An end of each spring 14 is in contact with the stopper bottom 9, to bias the presser 11 away from the stopper bottom 9 toward the front end of the housing 7.

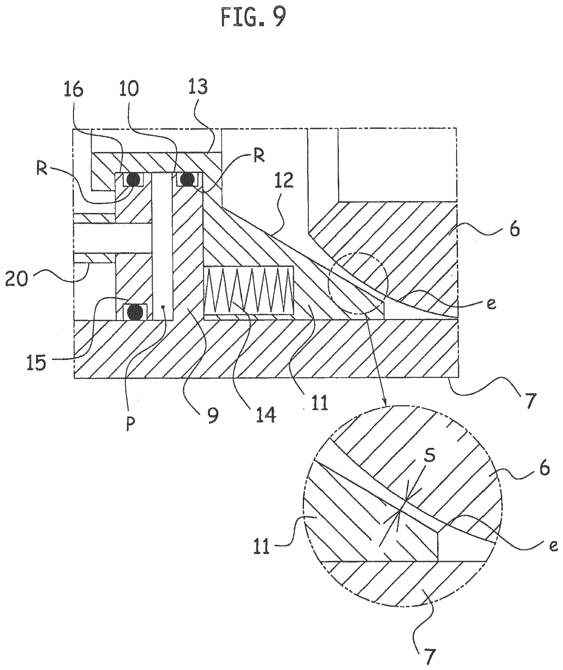

Behind the stopper bottom 9, there is an acting member 15 whose shape is substantially the same as that of the stopper bottom 9. The acting member 15 has a center hole 16 where an O-ring R is arranged. An outer circumference of the acting member 15 is also provided with an O-ring R, and therefore, the acting member 15 is airtightly in contact with an inner face of the housing 7 and the cylindrical presser end 13, to define a sealed chamber P between the stopper bottom 9 and the acting member 15 as illustrated in Fig. 9. The acting member 15 is coupled with an end of the cylindrical presser end 13 and is movable with the presser 11 in an axial direction.

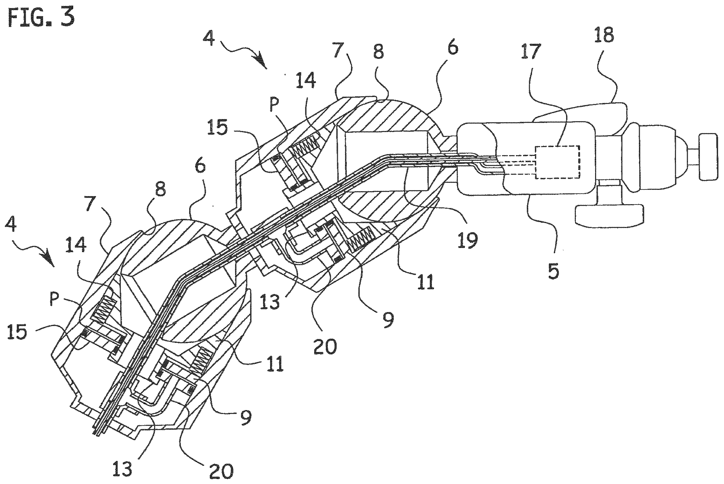

The three articulations 4 are connected in series with the proximal end of the housing 7 and the distal end of the ball 6 of the adjacent articulations 4 being connected to each other. The ball 6 of the foremost articulation 4 with respect to the base arm 3 is provided with the tool holder 5. As illustrated in Fig. 3, the tool holder 5 incorporates a valve 17 and is provided with a switch 18 to open and close the valve 17.

A flexible vinyl double pipe 19 is passed through the base arm 3, three articulations 4, and tool holder 5. The double pipe 19 is extended from an air pump (not illustrated) to the valve 17 of the tool holder 5.

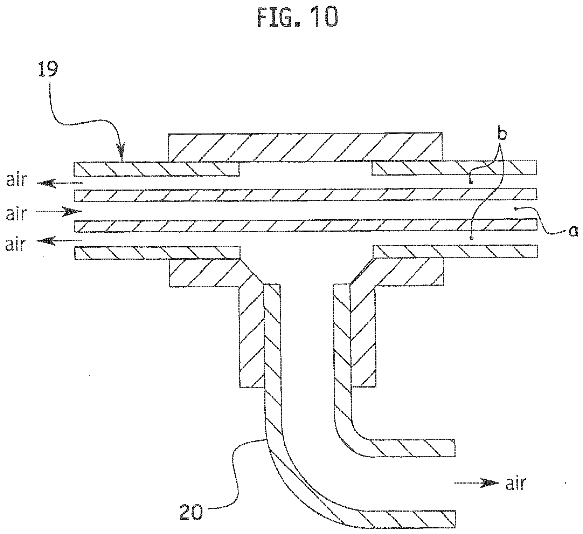

The double pipe 19 includes an inner passage a and an outer passage b that run in parallel with each other as illustrated in Fig. 10 and are connected to each other through the valve 17 as illustrated in Fig. 3. The air pump always supplies compressed air into the inner passage a of the double pipe 19. When the switch 18 of the tool holder 5 is pushed, the valve 17 opens to guide the compressed air from the inner passage a into the outer passage b. Namely, the compressed air oppositely flows in the inner passage a and outer passage b. In each of the articulations 4, a part of the outer passage b is branched into a branch passage 20, which is connected to the sealed chamber P.

The spring 14 as an elastic element, acting member 15, and sealed chamber P form the pressing mechanism stipulated in the claims.

Operation of the tool holding articulated arm 1 according to the embodiment will be explained.

In a normal state, the springs 14 in each articulation 4 bias the presser 11 toward the ball 6 and the presser 11 biases the ball 6. As a result, the ball 6 is held and nipped between the presser 11 and the front opening 8 of the housing 7. Since the presser 11 has a cup shape, a front circumferential edge e of the presser 11 linearly and firmly comes into contact with the surface of the ball 6, to create a large pressure between the edge e and the ball 6.

As a result, the ball 6 is strongly fixed by frictional force between the presser 11 and the front opening 8 of the housing 7. Namely, the balls 6 of the three articulations 4 no longer rotate, and therefore, the articulations 4 keep the fixed states . This configuration of holding the ball 6 between the presser 11 and the front opening 8 of the housing 7 creates a strong fixing force at each articulation 4 even with a small operating force of each spring 14.

To release the fixed state of each articulation 4 and optionally move the tool holding articulated arm 1, the switch 18 of the tool holder 5 is pushed. During an interval in which the switch 18 is being pushed, the valve 17 is open to pass compressed air from the inner passage a to the outer passage *b* of the double pipe 19. From the outer passage b through the branch passages 20, the compressed air is supplied into the sealed chambers P of the articulations 4.

When the compressed air is supplied into the sealed chamber P in each articulation 4, the acting member 15 moves together with the presser 11 in a direction away from the stopper bottom 9 against the force of the springs 14. This results in producing a slight gap S between the presser 11 and the ball 6 as illustrated in Fig. 9, to allow the ball 6 to freely rotate. The balls 6 of the three articulations 4 become simultaneously rotatable, and therefore, the articulations 4 as a whole become flexible to optional positions. The width (diameter) of the hollow of the ball 6 is sufficiently large compared to that of the double pipe 19, and therefore, no large stress is exerted on the double pipe 19 even if the articulations 4 are flexed to large angles.

The supply of compressed air into the double pipe 19 may be adjusted so that proper friction is maintained between the presser 11 and the ball 6 at each articulation 4 when flexing the tool holding articulated arm 1, instead of creating the clear gap S in each articulation 4 as illustrated in Fig. 9.

To stop the supply of compressed air into the sealed chambers P, the switch 18 is released. This results in reducing pressure in the sealed chambers P, and therefore, the springs 14 push the pressers 11, which again push the balls 6 to fix the articulations 4 in states when the switch 18 has been released.

According to the embodiment, the double pipe 19 alone realizes two opposing flows of compressed air in the tool holding articulated arm 1. This configuration reduces the number of pipes in the arm 1 and simplifies the internal structure of each articulation 4.

According to the embodiment, the articulations 4 are normally stationary with the bias of the springs 14, and only when required, are released from the stationary state with the use of compressed air supplied from a pump. This configuration is able to keep the articulations 4 immobile even if a contingency such as a blackout occurs.

As mentioned above, the tool holding articulated arm according to the first aspect of the present invention holds the ball between the presser and the front opening of the housing in each joint of the arm. This aspect is able to apply a large fixing force to each joint with a small operating force of the presser.

The tool holding articulated arm according to the second aspect of the present invention employs the cup-shaped presser so that the circumferential edge of the presser comes into linear contact with the surface of the ball. This aspect is able to exert a strong fixing force from the presser onto the surface of the ball.

The tool holding articulated arm according to the third aspect of the present invention always presses and fixes the pressers to the balls with mechanical pressing force of the springs, and only when required, supplies a gas into the sealed chambers to release the pressing of the pressers on the balls . This aspect is able to maintain the fixed state of the joints even if a contingency such as a blackout occurs.

The tool holding articulated arm according to the fourth aspect of the present invention is capable of fixing and releasing a plurality of the joints with a single piece of the double pipe. This aspect is able to simplify the internal structure of each joint.

## Claims

1. A medical tool holding articulated arm comprising:
a plurality of articulations (4) each including:
a ball (6) having a spherical surface;
a housing (7) accommodating the ball (6) and having a front opening (8) whose diameter is smaller than the diameter of the ball and a stopper bottom (9) opposing the front opening; and
a presser (11) arranged between the stopper bottom (9) and the ball (6) and moved toward and away from the ball by a pressing mechanism (14, 15, P), wherein the plurality of articulations (4) are connected in series with corresponding ends of the housing (7) and ball (6) of the adjacent articulations; and
a tool holder (5) for holding a tool attached to the ball of a foremost one of the plurality of articulations (4);
wherein the pressing mechanism (14, 15, P) includes:
a spring (14) arranged between the stopper bottom (9) and the presser (11) to bias the presser (11) toward the ball (6);
an acting member (15) coupled with a part of the presser (11) behind the stopper bottom (9); and
a sealed chamber (P) defined between the acting member and the stopper bottom,
wherein the acting member (15) is moving together with the presser away from the ball when a gas is supplied into the sealed chamber;
the tool holding articulated arm being **characterized by**
a double pipe (19) being flexible and passing through the plurality of articulations, the double pipe including an inner passage (a) and an outer passage (b) so that
a compressed gas is supplied in one direction into the inner passage, is turned at an end of the inner passage into the outer passage, and is guided in the opposite direction through the outer passage;
a valve (17) arranged at the end of the inner passage and controlled to open and close the flow of the compressed gas; and
a branch passage (20) branched from the outer passage in each of the plurality of articulations (4) and connected to the sealed chamber (P).

2. The tool holding articulated arm of claim 1, **characterized in that**
the presser (11) has a cup shape having a circumferential edge (e) that is brought into linear contact with the surface of the ball (6).

## Patentansprüche

1. Medizinischer gelenkiger Werkzeughaltearm, mit:
mehreren Gelenken (4), die jeweils aufweisen:
eine Kugel (6) mit einer Kugeloberfläche;
ein Gehäuse (7), das die Kugel (6) aufnimmt und eine vordere Öffnung (8), deren Durchmesser kleiner als der Durchmesser der Kugel ist, und einen Boden mit Haltefunktion (9) gegenüberliegend zu der vorderen Öffnung hat; und
einen Drücker (11), der zwischen dem Boden mit Haltefunktion (9) und der Kugel (6) angeordnet ist und durch einen Drückmechanismus (14, 15, P) zu der Kugel hin und von dieser weg bewegbar ist, wobei die mehreren Gelenke (4) in Reihe mit entsprechenden Enden des Gehäuses (7) und der Kugel (6) der benachbarten Gelenke verbunden sind; und
einem Werkzeughalter (5) zum Halten eines Werkzeugs, das an der Kugel eines vorderen Gelenks der mehreren Gelenke (4) angebracht ist;
wobei der Drückmechanismus (14, 15, P) aufweist:
eine Feder (14), die zwischen dem Boden mit Haltefunktion (9) und dem Drücker (11) zum Vorspannen des Drückers (11) in Richtung zu der Kugel (6) angeordnet ist;
ein Aktivierungselement (15), das mit einem Teil des Drückers (11) hinter dem Boden mit Haltefunktion (9) verbunden ist; und
eine abgedichtete Kammer (P), die zwischen dem Aktivierungselement und dem Boden mit Haltefunktion gebildet ist,
wobei das Aktivierungselement (15) sich zusammen mit dem Drücker von der Kugel weg bewegt, wenn Gas in die abgedichtete Kammer eingeführt wird;
wobei der gelenkige Werkzeughaltearm **gekennzeichnet ist durch** eine Doppelleitung (19), die flexibel ist und durch die mehreren Gelenke verläuft, wobei die Doppelleitung einen inneren Durchlass (a) und einen äußeren Durchlass (b) aufweist, derart, dass
ein komprimiertes Gas in dem inneren Durchlass in einer Richtung eingeführt wird, an einem Ende des inneren Durchlasses in den äußeren Durchlass gelenkt und in der entgegengesetzten Richtung durch den äußeren Durchlass geführt wird;
ein Ventil (17), das an dem Ende des inneren Durchlasses angeordnet und zum Öffnen und Schließen für das Strömen des komprimierten Gases steuerbar ist; und
einen Verzweigungsdurchlass (20), der von dem äußeren Durchlass in jedem der mehreren Gelenke (4) abzweigt und mit der abgedichteten Kammer (P) verbunden ist.

2. Gelenkiger Werkzeughaltearm nach Anspruch 1, **dadurch gekennzeichnet, dass** der Drücker (11) eine Becherform mit einem Umfangsrand (e) hat, der mit der Oberfläche der Kugel (6) geradlinig in Kontakt gebracht wird.

## Revendications

1. Bras articulé de support d'outil médical comprenant :
une pluralité d'articulations (4) comprenant chacune :
une bille (6) présentant une surface sphérique ;
un logement (7) réceptionnant la bille (6) et présentant une ouverture avant (8) dont le diamètre est inférieur au diamètre de la bille et un fond d'arrêt (9) opposé à l'ouverture avant ; et
un dispositif de pression (11) disposé entre le fond d'arrêt (9) et la bille (6) et déplacé vers et à l'opposé de la bille grâce à un mécanisme de pression (14, 15, P), où la pluralité des articulations (4) sont connectées en série aux extrémités correspondantes du logement (7) et de la bille (6) des articulations adjacentes ; et
un support d'outil (5) pour soutenir un outil fixé à la bille de la plus proche de la pluralité des articulations (4) ;
où le mécanisme de pression (14, 15, P) comprend :
un ressort (14) disposé entre le fond d'arrêt (9) et le dispositif de pression (11) pour incliner le dispositif de pression (11) vers la bille (6) ;
un élément d'actionnement (15) couplé à une partie du dispositif de pression (11) derrière le fond d'arrêt (9) ; et
une chambre hermétiquement fermée (P) définie entre l'élément d'actionnement et le fond d'arrêt,
où l'élément d'actionnement (15) se déplace conjointement au dispositif de pression à l'opposé de la bille lorsqu'un gaz est alimenté dans la chambre hermétiquement fermée ;
le bras articulé de support d'outil **caractérisé par**
une double canalisation (19) souple et passant à travers la pluralité des articulations, la double canalisation comprenant un passage interne (a) et un passage externe (b) de sorte que
un gaz comprimé est alimenté dans un sens dans le passage interne, est tourné à une extrémité du passage interne dans le passage externe, et est guidé dans le sens opposé à travers le passage externe ;
une vanne (17) disposée à l'extrémité du passage interne et régulée pour ouvrir et fermer l'écoulement du gaz comprimé ; et
un passage ramifié (20) ramifié depuis le passage externe dans chacune de la pluralité des articulations (4) et connecté à la chambre hermétiquement fermée (P).

2. Bras articulé de support d'outil selon la revendication 1, **caractérisé en ce que**
le dispositif de pression (11) présente une forme de coupe ayant un bord circonférentiel (e) qui est porté en contact linéaire avec la surface de la bille (6).
